# EUROPÄISCHE PATENTSCHRIFT

(11) **EP 4 155 288 B1**
(45) Veröffentlichungstag und Bekanntmachung des Hinweises auf die Patenterteilung: **08.04.2026**
(21) Anmeldenummer: 21198950.4
(22) Anmeldetag: 24.09.2021
(51) Int. Cl.: C07C 45/50, C07C 47/02

(54) **VERFAHREN ZUR VERBESSERTEN STEUERUNG DER ISOMERENVERHÄLTNISSE IN HYDROFORMYLIERUNGEN**
METHOD FOR IMPROVED CONTROL OF ISOMERIC RATIOS IN HYDROFORMYLATIONS
PROCÉDÉ DE COMMANDE AMÉLIORÉE DES RAPPORTS D'ISOMÈRES DANS LES HYDROFORMYLATIONS

(43) Veröffentlichungstag der Anmeldung: 29.03.2023
(73) Patentinhaber: OXEA GmbH, 40789 Monheim am Rhein NRW (DE)
(72) Erfinder: Balzarek, Christoph, 47803 Krefeld (DE); Hüttermann, Lars, 45899 Gelsenkirchen (DE); Johnen, Leif, 46286 Dorsten (DE); Meier, Gregor, 47057 Duisburg (DE); Musenbrock, Marcel, 40629 Düsseldorf (DE); Oeljeklaus, Julian, 45239 Essen (DE); Stefan, Alexander, 40233 Düsseldorf (DE); Vogelsang, Dennis, 48249 Dülmen (DE)
(74) Vertreter: Michalski Hüttermann & Partner Patentanwälte mbB

(56) Entgegenhaltungen:
- WO-A1-2009/035204

## Beschreibung

Die vorliegende Erfindung betrifft Verfahren zur Hydroformylierung von 1-Olefinen an einem rhodiumhaltigen Komplexkatalysator umfassend eine Mischung phosphorhaltiger organischer Komplexliganden unter Zutritt von Wasserstoff und Kohlenmonoxid, wobei die Umsetzung in einem Lösemittel ausgesucht aus der Gruppe der Lösemittel mit einem Siedepunkt von größer oder gleich 180°C und kleiner oder gleich 250°C, mit einer Rhodiumkonzentration von größer oder gleich 50 ppm und kleiner oder gleich 250 ppm an Katalysatorkomplexen mit mindestens zwei unterschiedlichen Komplexliganden ausgewählt aus der Gruppe bestehend aus Arylphosphinen und di- oder tri-Cycloalkylphosphinen durchgeführt wird. Des Weiteren betrifft die vorliegende Erfindung die Verwendung des erfindungsgemäßen Verfahrens im Rahmen einer zweistufigen Hydroformylierungs-Reaktionskaskade.

In Hydroformylierungsreaktionen werden Olefine mittels einer Synthesegasmischung aus Kohlenmonoxid und Wasserstoff in Gegenwart eines mit organischen Liganden komplexierten Metalls zu Aldehyden mit einem im Vergleich zum Olefin zusätzlichen Kohlenstoff umgesetzt. Dieses Reaktionsprinzip wurde im letzten Jahrhundert von Otto Roelen in Deutschland entwickelt und bildet eine grundlegende Reaktion im Bereich der homogenen Katalyse. Die erhältlichen Aldehyde können in weiteren Verarbeitungsschritten beispielsweise zu Carbonsäuren oxidiert oder zu Alkoholen hydriert oder anderweitig umgesetzt werden. Die Aldehyde selbst und die weiteren Reaktionsprodukte bilden wichtige industrielle Ausgangsstoffe und werden im großen Umfang beispielsweise als Lösungsmittel, Zusatzstoff, Rohstoff für Weichmacher und Schmierstoff verwendet.

Das Hydroformylierungsverfahren ist bzgl. der Regioselektivität an sich unspezifisch und liefert für 1- oder alpha-Olefine ein Gemisch aus linearen (n-) und verzweigten (iso)-Produktaldehyden. Mangels industrietauglicher alternativer Syntheserouten für isoselektive Reaktionen wurde der prinzipielle Erhalt von Isomerenmischung in dieser großtechnischen Reaktion in Kauf genommen. Dieser Kompromiss kann darin begründet liegen, dass aus chemischer Sicht die stereoselektive Hydroformylierung an der C2-Kohlenstoffposition herausfordernd ist, da unsubstituierte, lineare 1-Olefine keine elektronischen oder sterischen Vorzugsmerkmale aufweisen. Das erhältliche Isomerenverhältnis ist eine komplexe Funktion der vorherrschenden Reaktionsbedingungen, wobei für den eingesetzten Katalysator, und hier insbesondere der Ausbildung der Ligandensphäre des Katalysators, ein großer Einfluss auf die isomere Produktzusammensetzung beigemessen wird. Der Großteil des industriellen Interesses lag in den letzten Jahren auf der Prozessoptimierung in Richtung einer Ausbeutesteigerung für n-Aldehyde. Erst in der letzten Zeit hat sich ein verstärkter Bedarf an den entsprechenden verzweigten Aldehyden entwickelt, wobei neben der reinen Stereoselektivität natürlich auch die Wirtschaftlichkeit der gesamten Umsetzung im Sinne hoher Selektivitäten und ausreichender Umsätze als Randbedingungen berücksichtigt werden müssen.

Auch in der Patentliteratur finden sich eine Vielzahl an Verfahrensführungen, welche für Hydroformylierungsreaktionen speziellen Einfluss auf das Isomerenverhältnis ermöglichen sollen.

So offenbart beispielsweise die WO 2013 181 188 A1 ein Verfahren zur Herstellung von Aldehyden, umfassend: a) Kontaktieren einer Katalysatorzusammensetzung und eines ersten Olefins unter Hydroformylierungsbedingungen, um eine Katalysatorligandenzusammensetzung herzustellen; und b) Kontaktieren eines zweiten Olefins, von Wasserstoff und Kohlenmonoxid in Gegenwart der Katalysatorligandenzusammensetzung, um Aldehyde zu erzeugen, wobei das zweite Olefin Propylen ist, wobei das erste Olefin eine längere Kohlenstoffkette aufweist als das zweite Olefin, und wobei die Katalysatorligandenzusammensetzung Tris(3-pyridyl)phosphin, einen magnesiumzentrierten Tetraphenylporphyrinkoordinationskomplex und einen Liganden umfasst, der in situ durch Einschub des ersten Olefins in eine Rhodiumcarbonylbindung gebildet wird.

In einem weiteren Patentdokument, der EP 3 156 127 A1 werden Katalysatorzusammensetzungen beschrieben. Die Katalysatorzusammensetzung umfassen: spezielle einzähnige Phosphitliganden; spezielle einzähnige Phosphinliganden; und einen Übergangsmetallkatalysator, der durch die folgende Formel 3 dargestellt ist:

**M(L¹)ₓ(L²)_{y}(L³)_{z}**

wobei der Gesamtgehalt des gesamten Liganden einschließend den einzähnigen Phosphitliganden und den einzähnigen Phosphinliganden 1 bis 33 mol, basierend auf 1 mol des Übergangsmetallkatalysators, ist, wobei die Liganden des Katalysators R1, R2, R3, R'1, R'2 und R'3 jeweils unabhängig darstellen: eine substituierte oder unsubstituierte Cycloalkyl- oder Cycloalkenylgruppe mit 5 bis 20 Kohlenstoffatomen; oder eine substituierte oder unsubstituierte Arylgruppe mit 6 bis 36 Kohlenstoffatomen; und wenn R1, R2, R3, R'1, R'2 und R' 3 durch einen Substituenten substituiert sind, der Substituent Nitro (-NO₂), Fluor (-F), Chlor (-CI), Brom (-Br) oder eine Alkylgruppe mit 1 bis 20 Kohlenstoffatomen ist; wobei M ausgewählt aus der Gruppe bestehend aus Cobalt (Co), Rhodium (Rh), Iridium (Ir), Ruthenium (Ru), Eisen (Fe), Nickel (Ni), Palladium (Pd), Platin (Pt) und Osmium (Os) ist; L¹, L² und L³ jeweils unabhängig eines ausgewählt aus der Gruppe bestehend aus Wasserstoff, Carbonyl (CO), Cyclooctadien, Norbornen, Chlor, Triphenylphosphin (TPP) und Acetylacetonato (AcAc) darstellen, und x, y und z jeweils unabhängig 0 bis 5 darstellen, mit der Maßgabe, dass nicht alle x, y und z null sind, wobei der Gehalt jedes des einzähnigen Phosphitliganden und des einzähnigen Phosphinliganden 0,5 bis 32,5 mol ist, basierend auf 1 mol des Übergangsmetallkatalysators, wobei ein Mischungsverhältnis des einzähnigen Phosphitliganden und des einzähnigen Phosphinliganden 5:1 bis 1:5 ist, auf Gewichtsbasis.

In der WO 2009/035204 A1 wird eine Katalysatorzusammensetzung beschrieben, die einen Triphenylphosphinliganden, einen monodentaten Phosphinliganden, einen monodentaten Phosphinoxidliganden und einen Übergangsmetallkatalysator und ein Hydroformylierungsverfahren unter Verwendung desselben umfasst. Bei dem Hydroformylierungsverfahren unter Verwendung der Katalysatorzusammensetzung gemäß der vorliegenden Erfindung kann die hohe katalytische Aktivität erhalten werden und die Selektivität (n/iso-Selektivität) in Bezug auf Normal- oder Isoaldehyd kann wünschenswert gesteuert werden.

Weiterhin beschreibt die WO2009 035 204 A1 eine Katalysatorzusammensetzung, die einen Triphenylphosphinliganden, einen einzähnigen Phosphinliganden, einen einzähnigen Phosphinoxidliganden und einen Übergangsmetallkatalysator enthält, sowie ein Hydroformylierungsverfahren unter Verwendung desselben.

Derartige aus dem Stand der Technik bekannte Lösungen können noch weiteres Verbesserungspotential bieten. Dies bezieht sich insbesondere auf die Steuerung des gewünschten Isomerenverhältnisses unter Beibehaltung der Randbedingung eines hohen Umsatzes und einer hohen Ausbeute.

Es ist daher die Aufgabe der vorliegenden Erfindung, die aus dem Stand der Technik bekannten Nachteile zumindest teilweise zu überwinden. Es ist insbesondere die Aufgabe der vorliegenden Erfindung ein Verfahren bereitzustellen, welches mit hohen Umsätzen und Ausbeuten eine Steuerung des Isomerenverhältnisses, und insbesondere die Darstellung eines hohen Anteils an iso-Aldehyden, ermöglicht. Des Weiteren ist es die Aufgabe der vorliegenden Erfindung, eine effiziente Verwendung des Verfahrens bereitzustellen, wobei durch die Ankopplung des erfindungsgemäßen Verfahrens an einen vorgelagerten Prozessschritt eine verbesserte Gesamt-Verfahrensführung zur flexiblen Herstellung unterschiedlicher Mengen an Aldehyd-Isomeren ermöglicht wird.

Die Lösung der Aufgabe erfolgt durch die Merkmale der unabhängigen Ansprüche, gerichtet auf das erfindungsgemäße Verfahren sowie die erfindungsgemäße Verwendung des Verfahrens im Rahmen einer mehrschrittigen Herstellung. Bevorzugte Ausgestaltungen der Erfindung sind in den Unteransprüchen, in der Beschreibung oder den Figuren angegeben, wobei weitere in den Unteransprüchen, in der Beschreibung oder den Figuren beschriebene oder gezeigte Merkmale einzeln oder in einer beliebigen Kombination einen Gegenstand der Erfindung darstellen können, solange sich aus dem Kontext nicht eindeutig das Gegenteil ergibt.

Erfindungsgemäß gelöst wird die Aufgabe durch ein Verfahren zur Hydroformylierung von 1-Olefinen durch Umsetzung dieser an einem rhodiumhaltigen Komplexkatalysator umfassend eine Mischung phosphorhaltiger organischer Komplexliganden unter Zutritt von Wasserstoff und Kohlenmonoxid, wobei die Umsetzung in einem Druckbereich von größer oder gleich 0,5 MPa und kleiner oder gleich 5 MPa und in einem Lösemittel ausgesucht aus der Gruppe der Lösemittel mit einem Siedepunkt von größer oder gleich 180°C und kleiner oder gleich 250°C, mit einer Rhodiumkonzentration von größer oder gleich 50 ppm und kleiner oder gleich 250 ppm an Katalysatorkomplexen mit mindestens zwei unterschiedlichen Komplexliganden ausgewählt aus der Gruppe bestehend aus Arylphosphinen und tri-Cycloalkylphosphinen durchgeführt wird, wobei der Anteil der Cycloalkylphosphine an der gesamten Organophosphor-Ligandenmenge größer oder gleich 1 Mol% und kleiner oder gleich 67 Mol% ist und das molare Organophosphor-Liganden zu Rhodium-Verhältnis, ausgedrückt als molare Menge Organophosphor-Liganden dividiert durch molare Menge Rhodium, kleiner oder gleich 85 beträgt.

Überraschenderweise hat sich gezeigt, dass sich mittels der erfindungsgemäßen Verfahrensführung im Vergleich zu den im Stand der Technik üblicherweise erhältlichen Verfahrenskennzahlen, besonders große Mengen an verzweigten Aldehyden aus 1-Olefinen erzeugen lassen, wobei der hohe Anteil an iso-Aldehyden nicht durch eine Reduzierung des Olefin-Umsatzes oder durch eine verringerte Selektivität in Richtung der Aldehyde insgesamt in Kauf genommen werden muss. Die Reaktion kann also vorteilhafterweise mit hohen Geschwindigkeiten, hohen Umsätzen und mit sehr geringen Mengen unerwünschter Nebenprodukte gefahren werden, welches natürlich die Wirtschaftlichkeit des gesamten Verfahrens beeinflusst. Ohne durch die Theorie gebunden zu sein, scheint dies insbesondere auf eine vorteilhafte Kombination aus der spezifischen Ligandenumgebung des Katalysators, der eingesetzten Katalysatormenge insgesamt, in Kombination mit der Lösemittelauswahl zurückzuführen zu sein, welche zum einen ungehinderten Zutritt des Synthesegases ermöglicht und andererseits, durch die sterische Ausgestaltung der Katalysatorumgebung, ohne Produktivitätsverluste, zu hohen Mengen an iso-Aldehyden führt. Zwar ist der grundsätzliche Effekt einzelner Liganden auf das Isomerenverhältnis bekannt, dennoch wurde die Verschiebung zum iso-Isomer durch deutliche Einbußen in der Produktivität erkauft. Bisher ist es noch nicht gelungen Prozessbedingungen zu schaffen, welche einer effizienten Produktion unter gleichzeitiger Produktion hoher iso-Anteile gerecht wird. Des Weiteren ist das erfindungsgemäße Verfahren vorteilhaft, da die verbesserte Verfahrensführung in niedrigen Druckbereichen und mit relativ geringen Katalysatormengen erreichbar ist, welches zusätzlich zur verbesserten Wirtschaftlichkeit des gesamten Verfahrens in Hinblick auf die Investitions- und die laufenden Kosten beiträgt.

Das erfindungsgemäße Verfahren ist ein Verfahren zur Hydroformylierung von 1-Olefinen. 1- oder aber auch alpha-Olefine sind substituierte oder nicht substituierte aliphatische oder aromatische Kohlenwasserstoffe, welche zumindest eine endständige Doppelbindung in der 1-Position des Kohlenwasserstoffs aufweisen. Die Doppelbindung ist nicht in ein aromatisches System eingebunden. Mögliche Kohlenstoffzahlen der 1-Olefine können beispielsweise bis zu 15, bevorzugt bis zu 10, des Weiteren bevorzugt bis zu 8 betragen. Es können auch Mischungen unterschiedlicher 1-Olefine zur Umsetzung gebracht werden, wobei jedes der Olefine dann entsprechend eine endständige Doppelbindung aufweist. Mögliche Vertreter dieser Gruppe können beispielsweise Ethen, Propen, 1-Buten, 1-Penten, 1-Hexen, 1-Octen oder aber auch Styrol sein. Die entsprechenden Olefin-Edukte können an andere Stellen des Olefingerüstes noch weitere funktionelle Gruppen tragen, soweit diese nicht die Durchführung der erfindungsgemäßen Hydroformylierung verhindern.

Die Hydroformylierung erfolgt durch Umsetzung der 1-Olefine an einem rhodiumhaltigen Komplexkatalysator umfassend eine Mischung phosphorhaltiger organischer Komplexliganden unter Zutritt von Wasserstoff und Kohlenmonoxid. Die Olefine werden hydroformyliert, d.h. sie werden unter Zutritt von Synthesegas aus im wesentlichen Wasserstoff und Kohlenmonoxid in einer Reaktionszone an einem Katalysator unter Umwandlung der olefinischen in eine Aldehydgruppe zur Reaktion gebracht. Der erhältliche Aldehyd weist ein C-Atom mehr auf als das Einsatzolefin. Das erfindungsgemäße Verfahren kann in jedem geeigneten Reaktionsgefäß durchgeführt werden. Zu geeigneten Reaktionsgefäßen gehören beispielsweise Gas-Sparged-Reaktoren, Reaktoren mit einem Flüssigkeitsüberlauf, Tankreaktoren mit einem Rührwerk, sogenannten Trickle-Bed-Reaktoren etc.. Die zugeführten Synthesegasmengen und die Zusammensetzung desselben können innerhalb weiter Bereiche schwanken. In typischer Weise können die Verhältnisse von Wasserstoff zu Kohlenmonoxid bei 0,5:1 bis zu 10:1, weiter bevorzugt im Bereich von 1:1 bis zu 6:1 liegen.

Die Reaktion der Olefine zu den Aldehyden erfolgt mittels einer Rhodium-Metallkatalyse, wobei das Rhodium nicht als solches, sondern mit organischen Liganden sowie Kohlenmonoxid und Wasserstoff komplexiert vorliegt und so das katalytisch aktive Zentrum darstellt. Die genaue Zusammensetzung des Komplexes, und hier insbesondere die Stöchiometrie der Liganden inklusive der Synthesegasbestandteile, ist eine Funktion der vorherrschenden Reaktionsbedingungen. Zur Herstellung des aktiven Katalysators wird üblicherweise ein Rhodiumsalz in die Reaktionszone eingebracht und erfährt in dieser die Umwandlung in den eigentlich aktiven Katalysatorkomplex. Es ist aber auch möglich, dass der Katalysator unter ähnlichen Reaktionsbedingungen an einem anderen Ort, außerhalb der Reaktionszone, präformiert, d.h. in die aktive Spezies umgewandelt wird. Als nicht präformierte Rhodiumkomponenten können beispielsweise Rhodiumverbindungen eingesetzt werden, welche ausgesucht sind aus der Gruppe bestehend aus Rhodium(I)dicarbonylacetonylacetonat, Rhodium(II)2-ethylhexanoat, Rhodium(II)acetat, Rhodium(0)carbonyle (z.B. Rh₆(CO)I₆, Rh₄(CO)I₂), HRh(CO)(Ph₃P)₃, wobei Ph eine Phenylgruppe darstellt. Es können auch Mischungen zweier oder mehrerer dieser Rhodiumsalze eingesetzt werden. Es wurde gefunden, dass bevorzugt Rhodium-2-ethylhexanoat eingesetzt werden kann.

Der aktive Katalysatorkomplex weist in der Reaktionszone auf jeden Fall phosphorhaltige organische Komplexliganden in seiner Koordinationssphäre auf. Phosphorhaltige Komplexliganden sind dabei Kohlenwasserstoffe, bevorzugt aufweisend zyklische Gruppen, welche mindestens ein Phosphoratom im gesamten Kohlenwasserstoffgerüst aufweisen, wobei das Phosphoratom nicht in eine der zyklischen Gruppen eingebunden sein muss. Die phosphorhaltigen organischen Komplexliganden können beispielsweise der folgenden Formel entsprechen: 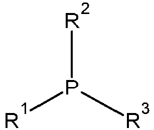 wobei die R¹, R² und R³ jeweils unabhängig voneinander aus der Gruppe bestehend aus substituierten oder nicht substitutierten Alkylgruppen mit 1 bis 20 C-Atomen; substituierte oder nicht substituierte Cycloalkylgruppen oder Cycloalkenylgruppen mit 5 bis 20 C-Atomen; substituierte oder nicht substituierte Arylgruppen mit 6 bis 36 C-Atomen; substituierte oder nicht substituierte Heteroalkylgruppen mit 1 bis 20 C-Atomen; substituierte oder nicht substituierte Heteroarylgruppen mit 4 bis 36 C-Atomen ausgesucht sein können, wobei im Fall der Substitution einer der Gruppen diese Substitution eine oder mehrere Atome aus der Gruppe bestehend aus N, O, und S aufweisen kann. Mögliche Vertreter dieser Gruppen sind beispielsweise die Triorganophosphine wie beispielsweise Triarylphosphin, Trialkylphosphin, Dialkylarylphosphin, Dicycloalkylarylphosphin, und Tricycloalkylphosphin.

Die Umsetzung wird in einem Druckbereich von größer oder gleich 0,5 MPa und kleiner oder gleich 5 MPa durchgeführt. Drücke im angegebenen Bereich können zu ökonomisch attraktiven Reaktionsgeschwindigkeiten unter erhöhten iso-Selektivitäten beitragen, wobei bei diesen relativ niedrigen Reaktionsdrücken die Kosten für die Reaktoranlage relativ moderat ausfallen, da der Reaktor dementsprechend weniger aufwendig ausgestaltet werden muss und die Notwendigkeit zusätzlicher Kompressor-Kapazitäten verringert ist.

Die Reaktion erfolgt in einem Lösemittel ausgesucht aus der Gruppe der Lösemittel mit einem Siedepunkt von größer oder gleich 180°C und kleiner oder gleich 250°C. Die Umsetzung der Olefine zu den Aldehyden erfolgt innerhalb eines inerten Lösemittels, welches während der Reaktion die oben erwähnten Rhodium-Komplexkatalysatoren als Lösungsmittel löst, wobei der Siedepunkt des Lösemittels unter Normdruck im oben angegebenen Bereich liegt. Mögliche Lösemittel können aus der Gruppe der Alkohole, Acetale oder Alkane mit einer Kettenlänger größer oder gleich C8 oder deren Mischungen ausgewählt sein. Mögliche Lösemittel, welche das erfindungsgemäße Siedepunktskriterium erfüllen, können beispielsweise aus der Gruppe bestehend aus 2-Ethylhexanol, 1-Octanol, 1-Decanol, höheren Aldehyd-Kondensationsprodukten einer oder dieser spezifischen Hydroformylierung oder Mischungen mindestens zweier Komponenten aus dieser Liste ausgesucht sein. Unter den höheren Aldehyd-Kondensationsprodukten einer Hydroformylierung wird dabei das Sumpfprodukt einer Hydroformylierung verstanden, welches sich im Laufe der Reaktion im Reaktor bildet. Diese höheren Kondensationsprodukte enthalten eine komplexe Mischung unterschiedlicher Bestandteile und werden auch als Dicköle bezeichnet. Die Aldehydprodukte sind selbst reaktiv und gehen langsam Kondensationsreaktionen ein. Diese Reaktion erfolgt selbst in Abwesenheit von Katalysatoren und wird durch die Prozessführung verursacht. Die flüssigen Kondensationsprodukte weisen naturgemäß höhere Siedepunkte als die Edukt-Aldehyde auf. Die Kondensationsprodukte können beispielsweise durch eine Aldol-Kondensation gebildet werden. Weitere Reaktionswege umfassen Tischshenko-Reaktionen, Umesterungen sowie Dismutationsreaktionen. Die Kondensationsprodukte sind Oligomere der Aldehyde und können noch weitere funktionale Gruppen wie Alkohol- oder Estergruppen tragen.

Die Hydroformylierung erfolgt mit einer Rhodiumkonzentration von größer oder gleich 50 ppm und kleiner oder gleich 250 ppm. Die Konzentration des in der Reaktionszone vorliegenden Rhodiums wird über das Gewichtsverhältnis von Rhodium zum Gesamtgewicht der Lösung in der Reaktionszone ausgedrückt. Mit dieser Konzentrationsangabe wird dabei auf das Verhältnis des reinen Metallgewichts (ohne Liganden) auf das Gesamtgewicht der Lösung inklusive eventuell weiterer Bestandteile, wie gelöste Liganden etc., abgestellt. Kleinere Konzentrationen können nachteilig sein, da die Reaktionsgeschwindigkeit dann zu gering ist. Höhere Konzentrationen können zur Reduktion des Anteils an iso-Aldehyd im Produkt und, bezogen auf die Kosten des Katalysatoreinsatzes, zu nur unterproportionalen Steigerungen in der Reaktionsgeschwindigkeit führen.

Die Katalysatorkomplexe weisen mindestens zwei unterschiedliche Komplexliganden ausgewählt aus der Gruppe bestehend aus Arylphosphinen und di oder tri-Cycloalkylphosphinen auf. Zur Steuerung des n/iso-Aldehydverhältnisses unter Beibehalt einer möglichst großen Reaktionsgeschwindigkeit hat sich die Zusammensetzung der Liganden als besonders wichtig herausgestellt. Hohe Umsätze und Selektivitäten können sich insbesondere unter bestimmten Mischverhältnissen von Arylphosphinen und di- oder tri-Cycloalkyl¬phos¬phinen einstellen. Arylphosphine sind beispielsweise Verbindungen der folgenden Formel: 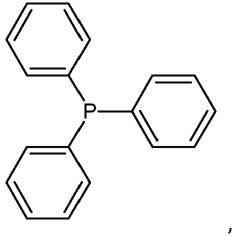 wobei die einzelnen Arylgruppen noch jeweils unabhängig voneinander substituiert vorliegen können. Unter der Gruppe der di- oder tri-Cycloalkyl¬phos¬phine werden beispielsweise folgenden C6-Cycloalkyl-Verbindungen gefasst: 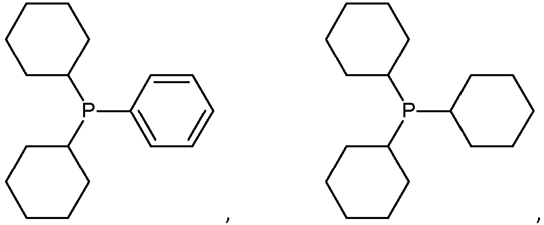 wobei die einzelnen Cykloalkyl- und/oder Arylgruppen jeweils unabhängig voneinander noch weitere, oben angegebene funktionelle Gruppen tragen können. Die Cycloalkyl-Gruppen können beispielsweise C3-C8 Cycloalkane, bevorzugt C4-C7 Cycloalkane sein.

Der Anteil der Cycloalkylphosphine an der gesamten Organophosphor-Ligandenmenge ist größer oder gleich 1 Mol% und kleiner oder gleich 67 Mol%. Zur erfindungsgemäßen Steuerung des verbesserten Isomerenverhältnisses unter Beibehalt einer hohen Umsetzungsgeschwindigkeit hat sich dieser enge Bereich des Cycloalkylphosphinverhältnisses als besonders geeignet herausgestellt. Der molare Anteil an Cycloalkylphosphin ergibt sich als Quotient aus molarer Cycloalkylphosphinmenge dividiert durch die Gesamtmenge an Organophosphorverbindungen, beispielsweise der Summe aus den Verbindungen mit den oben angegebenen Formeln aus der Gruppe der Aryl- und Cycloalkylphosphine. Die Cycloalkylphosphinmenge kann beispielsweise über ³¹P-Methoden quantitativ bestimmt werden. Die Liganden können dabei rein in der Reaktionszone vorgelegt oder aber auch schon über die Zugabe eines präformierten Metallkomplexes in die Reaktionslösung eingebracht werden. Zur Verdeutlichung, die Menge der Nicht-Organophosphor-Liganden des Rhodiums in der Reaktionszone, beispielsweise eingebracht durch die Katalysatorsalzbestandteile Acetat, Ethylhexanoat, CO etc. fließen nicht in die Berechnung des molaren Anteils der Cycloalkylphosphine ein.

Das molare Liganden zu Rhodium-Verhältnis, ausgedrückt als molare Menge Organophosphor -Liganden dividiert durch molare Menge Rhodium, beträgt kleiner oder gleich 85. Trotz der verringerten thermischen Stabilität der Cycloalkylphosphine hat es sich bewährt, mit nur einem relativ geringen Überschuss an Organophosphor-Liganden bezogen auf die molare Rhodiummenge zu fahren. Dieser Ligandenanteil ist in der Lage das benötigte n/iso-Verhältnis bereitzustellen, führt zu hohen Umsätzen und ist, überraschenderweise, über lange Produktionszeiträume in den angegebenen Lösemitteln stabil.

In einer bevorzugten Ausführungsform des Verfahrens kann die Hydroformylierung in einem Temperaturbereich von größer oder gleich 80°C und kleiner oder gleich 140°C durchgeführt werden. Innerhalb dieses Temperaturbereiches der Reaktionszone lassen sich ausreichende Reaktionsgeschwindigkeiten bereitstellen, wobei insbesondere auch bei diesen Umsätzen ein bevorzugtes Isomerenverhältnis erhalten wird, welches sich, verglichen mit den üblichen Stand der Technik Verfahren, durch einen höheren Anteil an iso-Isomeren auszeichnet.

Innerhalb einer weiter bevorzugten Ausgestaltung des Verfahrens kann das molare Verhältnis der Arylphosphin- zu den Cycloalkylphosphinliganden, ausgedrückt als molare Menge Arylphosphin- dividiert durch molare Menge Cycloalkylphosphinliganden, größer oder gleich 0,5 und kleiner oder gleich 75 betragen. Dieses Verhältnis zwischen Arylphosphin- und Cycloalkylphosphinliganden kann unter einer nur sehr geringen Reduzierung des Umsatzes ein deutlich erhöhtes iso-Isomerenverhältnis bereitstellen. In einer weiter bevorzugten Ausführungsform kann das Verhältnis größer oder gleich 15 und kleiner oder gleich 70, des Weiteren bevorzugt größer oder gleich 20 und kleiner oder gleich 60 betragen.

Innerhalb eines weiter bevorzugten Aspektes des Verfahrens kann das molare Verhältnis der Arylphosphinliganden zum Rhodium größer oder gleich 5 und kleiner oder gleich 75 betragen. Die Menge an Arylphosphinliganden kann neben dem Isomerenverhältnis auch deutlichen Einfluss auf die Produktivität der Gesamtreaktion haben. Innerhalb dieses molaren Verhältnisses lassen sich ausreichend hohe iso-Isomermengen mit hohen Umsätzen bereitstellen. Das molare Verhältnis kann des Weiteren bevorzugt größer oder gleich 35 und kleiner oder gleich 65, des Weiteren bevorzugt größer oder gleich 45 und kleiner oder gleich 55 betragen.

In einer weiter bevorzugten Ausführungsform des Verfahrens kann das molare Verhältnis der Cycloalkylphosphinliganden zu Rhodium größer oder gleich 1 und kleiner oder gleich 10 betragen. Die Menge an Cycloalkylphosphinliganden kann insbesondere einen deutlichen Einfluss auf das Isomerenverhältnis und insbesondere auf den iso-Anteil der gebildeten Aldehyde ausüben. Bei kleineren Verhältnissen ist der Einfluss der Cycloalkylphosphinliganden auf die Erhöhung des Iso-Aldehydanteils zu gering. Höhere Verhältnisse können nachteilig sein, da in diesen Fällen die Olefin-Umsatzraten deutlich reduziert werden können. Das molare Verhältnis kann des Weiteren bevorzugt größer oder gleich 2 und kleiner oder gleich 8, des Weiteren bevorzugt größer oder gleich 4 und kleiner oder gleich 6 betragen.

Innerhalb eines bevorzugten Aspektes des Verfahrens kann das Arylphosphin Triphenylphosphin sein. Der Einsatz von Triarylphosphin (TPP) als Arylphosphin kann zu besonders hohen Ausbeuten und zu besonders langen Standzeiten der Katalysatorlösung beitragen. TPP im Überschuss ist dabei insbesondere in der Lage, dass Rhodium in der Lösung zu stabilisieren. Zudem kann das TPP auch als Ligandenreservoir im Fall einer Schädigung des Cycloalkylphosphinliganden wirken.

Innerhalb eines bevorzugten Aspektes des Verfahrens kann das Cycloalkylphosphin Tricyclohexylphosphin sein. Insbesondere der Einsatz von Tricyclohexylphosphin kann zu einer besonders effizienten Verschiebung des Aldehyd-Isomerenverhältnisses in Richtung der iso-Isomere beitragen. Dies wird hochwahrscheinlich durch den erhöhten Raumanspruch des Liganden erreicht. Die Verschiebung des Isomerenverhältnisses zu iso-Aldheyden ergibt sich in den angegebenen Lösemitteln auch schon bei Konzentrationen, welche sich noch nicht nachteilig auf die mögliche, erreichbare Reaktionsgeschwindigkeit auswirken. Insofern kann dieser Ligand insbesondere effizienter beitragen als die Cycloalkylphosphine mit nur zwei Cycloalkylgruppen.

In einer weiter bevorzugten Ausführungsform des Verfahrens kann das 1-Olefin aus der Gruppe bestehend aus C3-C8 Olefinen oder Mischungen daraus ausgesucht sein. Gerade die mittleren Olefine können über die erfindungsgemäße Verfahrensführung ohne große Verluste im Umsatz verstärkt in Richtung der iso-Aldehydisomere umgesetzt werden. Ohne durch die Theorie gebunden zu sein, ergibt sich dieser Effekt bei den "mittleren" alpha-Olefinen durch die spezielle Ausrichtung des Olefins am Katalysatorkomplex, welcher durch die Ligandenzusammensetzung und das Lösemittel vorgegeben wird.

In einer bevorzugten Ausführungsform des Verfahrens kann das molare Verhältnis von Synthesegas zu 1-Olefin, ausgedrückt als (molare Menge H₂ + molare Menge CO) dividiert durch molare Menge 1-Olefin, größer oder gleich 1:1 und kleiner oder gleich 5:1 sein. Innerhalb dieser Relation zwischen Olefin und Synthesegas können in der beanspruchten Gruppe an Lösemitteln ausreichend hohe Konzentrationen an Reaktanden bereitgestellt werden, welche zusammen zu hohen Umsätzen und nur einer geringen Anzahl unerwünschter Nebenreaktion führen.

Des Weiteren erfindungsgemäß ist die Verwendung des erfindungsgemäßen Verfahrens zur Hydroformylierung von 1-Olefinen an einem Komplexkatalysator, wobei die Hydroformylierung in zwei Schritten durchgeführt wird, wobei innerhalb eines ersten Prozessschrittes die Umsetzung in Gegenwart eines rhodiumhaltigen Komplexkatalysators umfassend Arylphosphin- und ohne Cycloalkylphosphinliganden in einem Lösungsmittel ausgesucht aus der Gruppe der Alkohole, Acetale oder Alkane mit einer Kettenlänger größer oder gleich C10 oder deren Mischung durchgeführt wird, und wobei innerhalb eines zweiten Prozessschrittes ein weiteres Lösungsmittel mit einem Siedepunkt von größer oder gleich 180°C und kleiner oder gleich 250°C und zusätzlich tri-Cycloalkylphosphinliganden zu der Reaktionsmischung des ersten Prozessschrittes gegeben wird.

Überraschenderweise hat sich gezeigt, dass das erfindungsgemäße Verfahren sehr vorteilhaft im Rahmen einer zwei-schrittigen Hydroformylierungskaskade verwendet werden kann. In diesem nun zwei-schrittigen Verfahren können durch Einstellen der erfindungsgemäßen Reaktionsbedingungen nur im zweiten Schritt mehrere Vorteile erreicht werden. Durch das Ansetzen des zweiten Schrittes kann im Wesentlichen die Reaktionslösung in der Reaktionszone des ersten Schrittes mitverwendet werden, wobei das Einstellen auf die erfindungsgemäßen Bedingungen nur durch Zusatz von Liganden und Lösungsmittel bewerkstelligt werden kann. Aufwendige Trennoperation oder gar ein Austausch der gesamten Reaktionslösung können entfallen. Durch die Kopplung der beiden Verfahrensschritte kann zudem in Summe über beide Verfahrensschritte ein gewünschtes Isomerenverhältnis eingestellt werden, wobei sich die Lage des Verhältnisses auch über die Einstellung und die Lage des zweiten Schrittes ergeben. Zudem können vorteilhafterweise auch die im ersten Schritt gebildeten Dicköle mitverwendet werden und es kann ein Teil der Lösemittelzugabe mit sehr hohen Siedepunkten entfallen, da diese, zumindest zum Teil, schon in der Reaktionszone vorliegen.

In der erfindungsgemäßen Verwendung wird die Hydroformylierung in zwei Schritten durchgeführt, wobei innerhalb eines ersten Prozessschrittes die Umsetzung in Gegenwart eines rhodiumhaltigen Komplexkatalysators umfassend Arylphosphin- und ohne Cycloalkylphosphinliganden in einem Lösungsmittel ausgesucht aus der Gruppe der Alkohole, Acetale oder Alkane mit einer Kettenlänger größer oder gleich C10 oder deren Mischung durchgeführt wird. Der erste Prozessschritt der Kaskade erfolgt also nicht erfindungsgemäß unter Ausschluss von Cycloalkylliganden. In diesem Prozessschritt werden verstärkt n-Aldehyde gebildet. Es fallen innerhalb dieses Schrittes naturgemäß auch Dicköle an, welche aus der Eigenkondensation der produzierten Aldehyde stammen. Die Prozessbedingungen innerhalb dieses Schrittes können nicht denen des erfindungsgemäßen Verfahrens entsprechen, d.h., dass beispielsweise die Rhodiumkonzentrationen in der Reaktionszone höher liegen können als im erfindungsgemäßen Verfahren beansprucht.

Innerhalb eines zweiten Prozessschrittes wird ein weiteres Lösungsmittel mit einem Siedepunkt von größer oder gleich 180°C und kleiner oder gleich 250°C und zusätzlich Cycloalkylphosphinliganden zu der Reaktionsmischung des ersten Prozessschrittes gegeben. Durch die Zugabe des weiteren Lösungsmittels mit hohem Siedepunkt wird die Reaktionslösung des ersten Prozessschrittes auf die erfindungsgemäß Zusammensetzung des erfindungsgemäßen Verfahrens eingestellt. Durch die Verdünnung und die Zugabe der weiteren Ligandenspezies erhält man nun Reaktionsbedingungen, welche ohne nennenswerte Verluste in Umsatz und Produktivität einen höheren Anteil an iso-Isomeren erzeugt. Dies ist vorteilhafterweise möglich und führt durch die Verwendung eines Teils der Reaktionsumgebung des ersten Schrittes zu einer effizienten Nutzung der Reaktionslösung. Zudem kann je nach Isomerenbedarf der Anteil an erhältlichen Isomeren vorteilhafterweise über die Längen der einzelnen Prozessschritte gesteuert werden. Es ergibt sich in Summe ein synergistisch zusammenwirkender Gesamtprozess.

Innerhalb eines weiter bevorzugten Aspektes der Verwendung kann das Lösungsmittel im zweiten Prozessschritt aus der Gruppe bestehend aus Alkoholen mit einer Kettenlänge größer oder gleich C8, aus Acetalen mit einer Kettenlänge größer oder gleich C13, aus Alkanen mit einer Kettenlänger größer oder gleich C10, oder Mischungen mindestens zweier Komponenten aus dieser Gruppe ausgesucht sein. Diese Gruppe an zusätzlich eingesetzten Lösemitteln im zweiten Prozessschritt kann dazu beitragen, dass insbesondere für den zweiten Prozessschritt verbesserte Katalysator-Standzeiten bei hohen Umsätzen erhältlich sind.

Nach einer bevorzugten Charakteristik der Verwendung kann das Gewichtsverhältnis vom im zweiten Prozessschritt zugegebenen Lösungsmittel zur Lösungsmittelmenge des ersten Prozessschrittes größer oder gleich 4 und kleiner oder gleich 20 betragen. Zum Erhalt einer möglichst effizienten Lösungsmittelmischung, bestehend aus dem gebildeten hochsiedenden Lösemittel des ersten Prozessschrittes und dem im zweiten Prozessschritt zugegebenen hochsiedenden Lösemittel, hat sich oben angegebenes Mischungsverhältnis beider Lösemittel als besonders geeignet herausgestellt. Im zweiten Prozessschritt werden lange Katalysatorstandzeiten, hohe Umsätze und ein hoher iso-Isomerenanteil für die Aldehyde erhalten.

In einer weiter bevorzugten Ausführungsform der Verwendung kann die Konzentration der Arylphosphinliganden im ersten Prozessschritt bezogen auf das Gesamtgewicht der Prozesslösung größer oder gleich 10 Gew. % und kleiner oder gleich 30 Gew. % betragen. Zu besonders effizienten Einstellung der erfindungsgemäßen Ligandenverhältnisse im zweiten Prozessschritt hat sich diese Konzentration an Arylphosphinliganden im ersten Prozessschritt als besonders geeignet herausgestellt. Diese Konzentration führt zu einem stabilen Umsatz mit einer relativ geringen Katalysator-Deaktivierung im ersten Prozessschritt, ist aber gleichzeitig auch niedrig genug, um ohne zu viel des weiter zuzugebenden Katalysators in der zweiten Verfahrensstufe zu verschwenden. Es wird ein in Summe über beide Verfahrensstufen effizientes Gesamtverfahren erhalten, welches neben einer geeigneten Steuerung der n/iso-Verhältnisse auch insgesamt hohe Umsatzraten zeigt.

Innerhalb eines bevorzugten Aspektes der Verwendung kann die Konzentration des im zweiten Prozessschritt zugegebenen Cycloalkylphosphins bezogen auf das Gesamtgewicht der Prozesslösung größer oder gleich 0,01 Gew. % und kleiner oder gleich 1 Gew. % betragen. Für die Verfahrensökonomie und für eine effiziente Steigerung der iso-Anteile im Produkt hat es sich gezeigt, dass nur relativ geringe Mengen an Cycloalkylphosphin zur Reaktionslösung des zweiten Prozessschrittes hinzugegeben werden müssen. Insbesondere durch die Zugabe des Cycloalkylphosphins werden verstärkt iso-Aldehyde gebildet, wobei über die gewählten Reaktionsbedingungen ein im Vergleich zum ersten Prozessschritt starker Umsatzrückgang im zweiten Prozessschritt vermieden werden kann.

Innerhalb eines bevorzugten Aspektes der Verwendung kann die Gesamtmenge an Rhodium im ersten Prozessschritt zugegeben werden. Zur vereinfachten Verfahrensführung, zum Erhalt eines bevorzugten hohen iso-Verhältnisses der gebildeten Aldehyde und für hohe Umsätze hat es sich als geeignet herausgestellt, dass die Gesamtzugabe des metallischen Komplexkatalysators im Rahmen des ersten Prozessschrittes stattfindet. Dies ist erstaunlich, da im zweiten Prozessschritt aufgrund des geänderten Ligandenangebots sich erst ein Gleichgewicht einstellen muss, welches erwartungsgemäß bei Zusatz frischen Katalysators aufgrund der geänderten Gleichgewichtslage schneller passieren sollte. Dies ist überraschenderweise nicht der Fall.

Weitere Einzelheiten, Merkmale und Vorteile des Gegenstandes der Erfindung ergeben sich aus den Unteransprüchen sowie aus der nachfolgenden Beschreibung der Figuren und der zugehörigen Beispiele. Es zeigt die:
- Fig. 1: den iso-Aldehydanteil und die 1-Butenaufnahme einer Reaktionslösung mit 2-Ethylhexanol als Lösungsmittel als Funktion des molaren Tricyclohexylphosphin- /Rhodium-Verhältnisses;
- Fig. 2: den iso-Aldehydanteil und die 1-Butenaufnahme einer Reaktionslösung mit 2-Ethylhexanol als Lösungsmittel als Funktion des Tricyclohexylphosphin-Anteils an Organophosphor-Liganden;
- Fig. 3: den iso-Aldehydanteil und die 1-Butenaufnahme einer Reaktionslösung mit Rh-Triphenylphosphin-Katalysator nach Verdünnung mit 2-Ethylhexanol als Lösungsmittel, unter Abwesenheit von Tricyclohexylphosphin, als Funktion der Rhodium-Konzentration;
- Fig. 4: den iso-Aldehydanteil und die 1-Butenaufnahme einer Reaktionslösung mit Rh-Triphenylphosphin-Katalysator nach Verdünnung mit 2-Ethylhexanol als Lösungsmittel, und mit Tricyclohexylphosphin, als Funktion der Rhodium-Konzentration.

### Beispiele

Sämtliche Versuche wurden in einem Reaktor in Batch-Fahrweise mit einer präformierten Katalysatorphase (9 bar Synthesegasdruck (SynGas), Temperatur 120°C für 30 min) durchgeführt. Es erfolgte eine kontinuierliche Zugabe von 1-Buten/SynGas 1:1 mit 13 bar bei 120°C, wobei mit variierenden Reaktionszeiten von 20 min bis 2 h die Reaktion bis zu einem Umsatz von 25 g 1-Buten gefahren wurde. In sämtlichen Versuchen wurde die Katalysatorlösung unter inerten Bedingungen in den evakuierten Reaktor überführt und die Reaktion mit einer kontinuierlichen Zudosierung von 1-Buten und Synthesegas gestartet. Als Kennwerte wurden die resultierende zeitliche 1-Butenaufnahme (Systemproduktivität) und das iso/n-Verhältnis der C5-Aldehyde zur Bestimmung des 2-Methylbutanal (2-MB) Anteils verwendet.

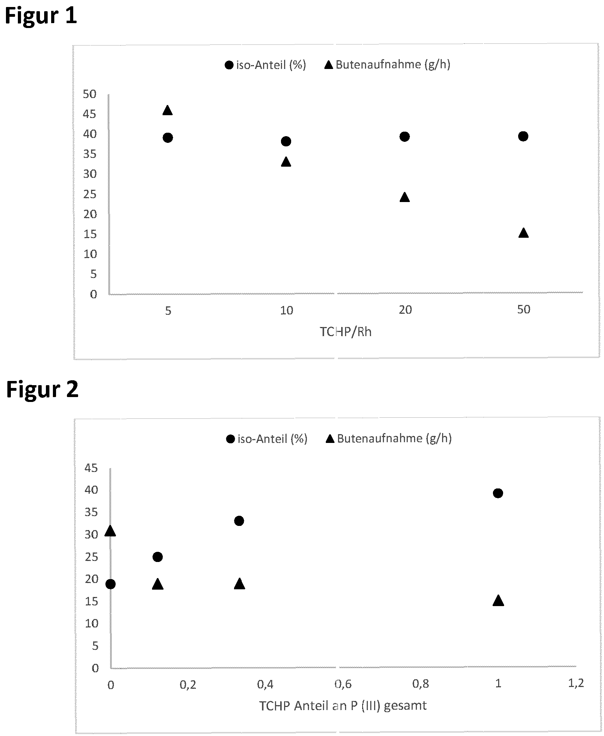

In der ersten Versuchsreihe wurde der Tricyclohexylphosphin (TCHP) Einfluss mit variierenden TCHP/Rh-Verhältnissen auf das iso/n-Verhältnis der gebildeten C5-Aldehyde und die 1-Butenaufnahme untersucht. Die Ergebnisse sind in der Tabelle 1 und in der Figur 1 dargestellt:

**Tabelle 1: Hydroformylierung von 1-Buten mit TCHP in 2-Ethylhexanol (2-EHol)**

| Exp. | LM | Rh (ppm) | TPP/Rh | TCHP/Rh | TCHP (Gew.%) | Umsatz % It. GC | iso:n | 1-Buten (g/h) |
|---|---|---|---|---|---|---|---|---|
| 1. | 2-EHol | 50 | 65 | 0 | 0 | 99 | 24:76 | 43 |
| 2. | 2-EHol | 50 | 0 | 5 | 0,07 | 94 | 39:61 | 46 |
| 3. | 2-EHol | 50 | 0 | 10 | 0,14 | 92 | 38:62 | 33 |
| 4. | 2-EHol | 50 | 0 | 20 | 0,27 | 91 | 39:61 | 24 |
| 5. | 2-EHol | 50 | 0 | 50 | 0,68 | 82 | 39:61 | 15 |

Aus der Tabelle 1 und Figur 1 wird ersichtlich, dass TCHP im Vergleich zu TPP den Anteil an 2-MB im Produkt steigert und damit zu Erhöhung des iso/n-Verhältnisses führt. Bereits mit einem geringen TCHP/Rh-Verhältnis wird ein positiver Effekt des Liganden auf die Bildung des verzweigten Aldehyds ausgeübt. Wahrscheinlich führt der hohe Raumanspruch des TCHP-Liganden und die resultierende Änderung der Geometrie des Rh-Komplexes und dessen Eigenschaften zur Änderung der Selektivität der 1-Buten-Hydroformylierung. Unter höheren TCHP/Rh-Verhältnissen sinkt jedoch auch die 1-Butenaufnahme und damit die Reaktionsgeschwindigkeit des Gesamtsystems. Die verringerte Olefin-Aufnahme kann wahrscheinlich auf die hohe Bindungsaffinität des TCHP-Liganden zum Rhodium zurückgeführt werden.

In einer weiteren Versuchsreihe wurde für das erfindungsgemäße Verfahren ein Ligandengemisch aus TCHP und Triphenylphosphin (TPP) eingesetzt. Falls vorhanden, wurde ein TCHP-Rh-Verhältnis von 50 vorgegeben und die TPP-Menge in der Reaktionslösung variiert. Die weiteren spezifischen Versuchsbedingungen sind oben angegeben. Die Ergebnisse sind in der Tabelle 2 dargestellt.

**Tabelle 2: Hydroformylierung von 1-Buten mit TCHP und TPP in 2-Ethylhexanol (2-EHol)**

| Exp. | LM | Rh (ppm) | TPP/Rh | TCHP/Rh | TCHP (Gew.%) | Umsatz % It. GC | iso:n | 1-Buten g/h |
|---|---|---|---|---|---|---|---|---|
| 1. | 2-EHol | 50 | 65 | 0 | 0 | 99 | 24:76 | 43 |
| 5. | 2-EHol | 50 | 0 | 50 | 0,68 | 82 | 39:61 | 15 |
| 6. | 2-EHol | 50 | 100 | 50 | 0,68 | 76 | 33:67 | 19 |
| 7. | 2-EHol | 50 | 360 | 50 | 0,68 | 79 | 25:75 | 19 |
| 8. | 2-EHol | 50 | 360 | 0 | 0 | 87 | 19:81 | 31 |

Die Figur 2 zeigt die Abhängigkeit des erhältlichen iso-Anteils und der Butenaufnahme als Funktion des TCHP-Anteils am durch die Liganden eingetragenen gesamten Phosphor(III). Äquivalent zum TCHP/Rh-Verhältnis findet man mit steigendem TCHP-Anteil einen höheren iso-Anteil im Produkt, jedoch verringern sich auch die Butenaufnahme und damit auch die Reaktionsgeschwindigkeit signifikant. Durch den gemeinsamen TPP- und TCHP-Ligandeneinsatz kann, unter spezifischen Konzentration- und Anteilsbeziehungen zwischen Liganden und Katalysator und den Liganden untereinander, die Reaktion unter hohen Umsätzen und unter hohen iso-Verhältnissen gefahren werden. Der TPP-Einsatz führt zu höheren Produktivitäten, welche auch bei höheren TPP/Rh-Verhältnissen weiterhin erhalten bleiben. Zugleich stabilisiert ein größerer TPP-Überschuss das Katalysatorsystem gegen Deaktivierung, sodass lange Prozesslaufzeiten der Katalysatorlösung erhältlich werden. Die Zugabe eines geringen TCHP-Überschusses im Verhältnis zu der molaren Rhodium-Menge ist dabei in der Lage, den 2-MB Anteil in der Reaktionslösung bei gleichbleibender Katalysatoraktivität zu erhöhen.

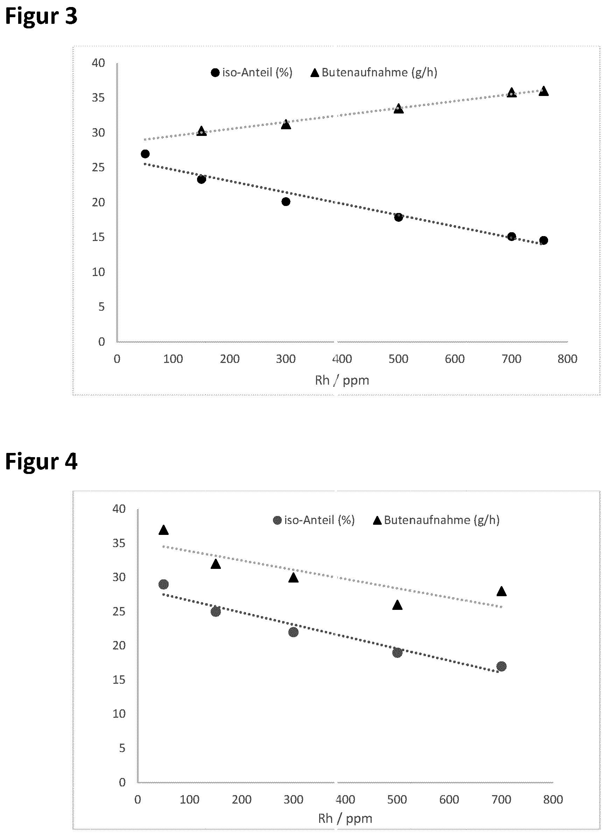

Die Figur 3 zeigt den iso-Aldehydanteil und die 1-Butenaufnahme einer Reaktionslösung mit Rh-Triphenylphosphin-Katalysator nach Verdünnung mit 2-Ethylhexanol als Lösungsmittel, unter Abwesenheit von Tricyclohexylphosphin, als Funktion der Rhodium-Konzentration. Das TPP-Rh-Verhältnis lag bei sämtlichen Versuchen bei 66. Aufgrund der Verdünnung der Katalysatorlösung mit 2-Ethylhexanol ergibt sich eine geringere Katalysatorkonzentration, welche dementsprechend in einem geringeren 1-Butenumsatz resultiert. Die linearen Regressionen über die beiden Parameter ergeben einen zu erwartenden, linearen Zusammenhang zwischen Butenaufnahme und iso-Anteil von der Katalysatorkonzentration bei Einsatz eines reinen TPP-Ligandensystems. Mit steigender Rh-Konzentration aber gleichbleibendem TPP/Rh-Verhältnis nimmt die Produktivität des Katalysatorsystems zu, wobei der iso-Anteil im Produkt durch den Einsatz eines reinen TPP-Ligandensystems abnimmt. Um dem sinkenden iso-Anteil entgegen zu wirken, und damit die Bildung von 2-MB zu steigern, wird im erfindungsgemäßen Verfahren dem Rh-TPP-Katalysatorsystem TCHP-Ligand zugegeben.

Die Figur 4 zeigt den iso-Aldehydanteil und die 1-Butenaufnahme einer Reaktionslösung mit gemischtem Rh-TPP-TCHP-Katalysator in einer Verdünnungsreihe mit 2-Ethylhexanol als Lösungsmittel als Funktion der Rhodium-Konzentration. Der Versuch entspricht also im Wesentlichen der Durchführung wie für die Figur 4 beschrieben, nur wird in dieser Reihe ein gemischtes Ligandensystem aus TPP und TCHP verwendet. Überraschenderweise findet man bei der Verwendung einer Ligandenmischung (TPP/Rh 69 und TCHP/Rh 2) sowohl einen Anstieg der Butenaufnahme wie auch einen Anstieg des iso-Aldehydanteils mit der Verdünnung sinkender Katalysatormetallkonzentration. Somit ist der Verlauf überraschenderweise fundamental anders als der Verlauf bei einem reinem TPP-Katalysatorsystem, wie unter Bezug auf die Figur 4 diskutiert. Dieser Zusammenhang zeigt deutlich, dass unter der Verwendung eines erfindungsgemäßen Ligandensystems aus zwei Liganden (TPP/TCHP) in einem Lösemittel, mit relativ geringen Katalysatorkonzentrationen und einem spezifischen Mengenverhältnis der beiden Liganden zueinander, sich überraschenderweise sowohl hohe Umsätze wie auch hohe iso-Aldehydanteile realisieren lassen. Die besonderen Vorteile des erfindungsgemäßen Verfahrens basieren auf dem spezifischen Mengenverhältnis der beiden Liganden. Zum einen reichen nur geringe molare Mengen an TCHP-Liganden bezogen auf das Rhodium-Metall, um durch den räumlich anspruchsvollen Liganden den iso-Anteil im Produkt zu erhöhen. Auch die Katalysatoraktivität ist mit diesem Liganden- und niedrigen TCHP/Rh-Verhältnis sehr hoch. Zum anderen wurde die molare Menge an TPP-Liganden bezogen auf das Rhodium-Metall so gewählt, dass das Rhodium-Liganden-Katalysatorsystem stabilisiert und die Katalysatoraktivität aufrechterhalten wird.

## Patentansprüche

1. Verfahren zur Hydroformylierung von 1-Olefinen durch Umsetzung dieser an einem rhodiumhaltigen Komplexkatalysator umfassend eine Mischung phosphorhaltiger organischer Komplexliganden unter Zutritt von Wasserstoff und Kohlenmonoxid, **dadurch gekennzeichnet, dass** die Umsetzung in einem Druckbereich von größer oder gleich 0,5 MPa und kleiner oder gleich 5 MPa und in einem Lösemittel ausgesucht aus der Gruppe der Lösemittel mit einem Siedepunkt von größer oder gleich 180°C und kleiner oder gleich 250°C, mit einer Rhodiumkonzentration von größer oder gleich 50 ppm und kleiner oder gleich 250 ppm an Katalysatorkomplexen mit mindestens zwei unterschiedlichen Komplexliganden ausgewählt aus der Gruppe bestehend aus Arylphosphinen und tri-Cycloalkylphosphinen durchgeführt wird, wobei der Anteil der Cycloalkylphosphine an der gesamten Organophosphor-Ligandenmenge größer oder gleich 1 Mol% und kleiner oder gleich 67 Mol% ist und das molare Organophosphor-Liganden zu Rhodium-Verhältnis, ausgedrückt als molare Menge Organophosphor-Liganden dividiert durch molare Menge Rhodium, kleiner oder gleich 85 beträgt.

2. Verfahren nach Anspruch 1, wobei die Hydroformylierung in einem Temperaturbereich von größer oder gleich 80°C und kleiner oder gleich 140°C durchgeführt wird.

3. Verfahren nach einem der vorhergehenden Ansprüche, wobei das molare Verhältnis der Arylphosphin- zu den Cycloalkylphosphinliganden, ausgedrückt als molare Menge Arylphosphin- dividiert durch molare Menge Cycloalkylphosphinliganden, größer oder gleich 0,5 und kleiner oder gleich 75 beträgt.

4. Verfahren nach einem der vorhergehenden Ansprüche, wobei das molare Verhältnis der Arylphosphinliganden zum Rhodium größer oder gleich 5 und kleiner oder gleich 75 beträgt.

5. Verfahren nach einem der vorhergehenden Ansprüche, wobei das molare Verhältnis der Cycloalkylphosphinliganden zu Rhodium größer oder gleich 1 und kleiner oder gleich 10 beträgt.

6. Verfahren nach einem der vorhergehenden Ansprüche, wobei das Arylphosphin Triphenylphosphin ist.

7. Verfahren nach einem der vorhergehenden Ansprüche, wobei das Cycloalkylphosphin Tricyclohexylphosphin ist.

8. Verfahren nach einem der vorhergehenden Ansprüche, wobei das 1-Olefin ausgesucht ist aus der Gruppe bestehend aus C3-C8 Olefinen oder Mischungen daraus.

9. Verfahren nach einem der vorhergehenden Ansprüche, wobei das molare Verhältnis von Synthesegas zu 1-Olefin, ausgedrückt als (molare Menge H₂ + molare Menge CO) dividiert durch molare Menge 1-Olefin, größer oder gleich 1:1 und kleiner oder gleich 5:1 ist.

10. Verwendung des Verfahrens nach einem der vorhergehenden Ansprüche zur Hydroformylierung von 1-Olefinen an einem Komplexkatalysator, **dadurch gekennzeichnet, dass** die Hydroformylierung in zwei Schritten durchgeführt wird, wobei innerhalb eines ersten Prozessschrittes die Umsetzung in Gegenwart eines rhodiumhaltigen Komplexkatalysators umfassend Arylphosphin- und ohne Cycloalkylphosphinliganden in einem Lösungsmittel ausgesucht aus der Gruppe der Alkohole, Acetale oder Alkane mit einer Kettenlänger größer oder gleich C10 oder deren Mischung durchgeführt wird, und wobei innerhalb eines zweiten Prozessschrittes ein weiteres Lösungsmittel mit einem Siedepunkt von größer oder gleich 180°C und kleiner oder gleich 250°C und zusätzlich tri-Cycloalkylphosphinliganden zu der Reaktionsmischung des ersten Prozessschrittes gegeben wird.

11. Verwendung nach Anspruch 10, wobei das Lösungsmittel im zweiten Prozessschritt ausgesucht ist aus der Gruppe bestehend aus Alkoholen mit einer Kettenlänge größer oder gleich C8, aus Acetalen mit einer Kettenlänge größer oder gleich C13, aus Alkanen mit einer Kettenlänger größer oder gleich C10, oder Mischungen mindestens zweier Komponenten aus dieser Gruppe.

12. Verwendung nach einem der Ansprüche 10 oder 11, wobei das Gewichtsverhältnis vom im zweiten Prozessschritt zugegebenen Lösungsmittel zur Lösungsmittelmenge des ersten Prozessschrittes größer oder gleich 4 und kleiner oder gleich 20 beträgt.

13. Verwendung nach einem der Ansprüche 10 bis 12, wobei die Konzentration der Arylphosphinliganden im ersten Prozessschritt bezogen auf das Gesamtgewicht der Prozesslösung größer oder gleich 10 Gew. % und kleiner oder gleich 30 Gew. % beträgt.

14. Verwendung nach einem der Ansprüche 10 bis 13, wobei die Konzentration des im zweiten Prozessschritt zugegebenen Cycloalkylphosphins bezogen auf das Gesamtgewicht der Prozesslösung größer oder gleich 0,01 Gew. % und kleiner oder gleich 1 Gew. % beträgt.

15. Verwendung nach einem der Ansprüche 10 bis 14, wobei die Gesamtmenge an Rhodium im ersten Prozessschritt zugegeben wird.

## Claims

1. Process for the hydroformylation of 1-olefins by reacting 1-olefins on a rhodium-containing complex catalyst comprising a mixture of phosphorus-containing organic complex ligands in the presence of hydrogen and carbon monoxide, **characterized in that** the reaction is carried out in a pressure range of greater than or equal to 0.5 MPa and less than or equal to 5 MPa, and in a solvent selected from the group of solvents having a boiling point of greater than or equal to 180°C and less than or equal to 250°C, with a rhodium concentration of greater than or equal to 50 ppm and less than or equal to 250 ppm on catalyst complexes with at least two different complex ligands selected from the group consisting of arylphosphines and tri-cycloalkylphosphines, wherein the proportion of cycloalkylphosphines in the total organophosphorus ligand amount is greater than or equal to 1 mol% and less than or equal to 67 mol% and the molar organophosphorus ligand to rhodium ratio, expressed as molar amount of organophosphorus ligand divided by molar amount of rhodium, is less than or equal to 85.

2. The process according to claim 1, wherein the hydroformylation is carried out in a temperature range of greater than or equal to 80°C and less than or equal to 140°C.

3. The process according to any one of the preceding claims, wherein the molar ratio of arylphosphine to cycloalkylphosphine ligands, expressed as molar amount of arylphosphine divided by molar amount of cycloalkylphosphine ligands, is greater than or equal to 0.5 and less than or equal to 75.

4. The process according to any one of the preceding claims, wherein the molar ratio of the arylphosphine ligands to rhodium is greater than or equal to 5 and less than or equal to 75.

5. The process according to any one of the preceding claims, wherein the molar ratio of cycloalkylphosphine ligands to rhodium is greater than or equal to 1 and less than or equal to 10.

6. The process according to any one of the preceding claims, wherein the arylphosphine is triphenylphosphine.

7. The process according to any one of the preceding claims, wherein the cycloalkylphosphine is tricyclohexylphosphine.

8. The process according to any one of the preceding claims, wherein the 1-olefin is selected from the group consisting of C3-C8 olefins or mixtures thereof.

9. The process according to any one of the preceding claims, wherein the molar ratio of synthesis gas to 1-olefin, expressed as (molar amount of H₂ + molar amount of CO) divided by molar amount of 1-olefin, is greater than or equal to 1:1 and less than or equal to 5:1.

10. Use of the process according to one of the preceding claims for the hydroformylation of 1-olefins on a complex catalyst, **characterized in that** the hydroformylation is carried out in two steps, wherein within a first process step the reaction is carried out in the presence of a rhodium-containing complex catalyst comprising arylphosphine ligands and without cycloalkylphosphine ligands in a solvent selected from the group consisting of alcohols, acetals or alkanes with a chain length greater than or equal to C10 or a mixture thereof, and wherein within a second process step a further solvent with a boiling point greater than or equal to 180°C and less than or equal to 250°C and additionally cycloalkylphosphine ligands are added to the reaction mixture of the first process step.

11. Use according to claim 10, wherein the solvent in the second process step is selected from the group consisting of alcohols having a chain length greater than or equal to C8, acetals having a chain length greater than or equal to C13, alkanes having a chain length greater than or equal to C10, or mixtures of at least two components of this group.

12. Use according to any one of claims 10 or 11, wherein the weight ratio of the solvent added in the second process step to the amount of solvent of the first process step is greater than or equal to 4 and less than or equal to 20.

13. Use according to any one of claims 10 to 12, wherein the concentration of the arylphosphine ligands in the first process step is greater than or equal to 10% by weight and less than or equal to 30% by weight based on the total weight of the process solution.

14. Use according to any one of claims 10 to 13, wherein the concentration of the cycloalkylphosphine added in the second process step is greater than or equal to 0.01% by weight and less than or equal to 1% by weight based on the total weight of the process solution.

15. Use according to any one of claims 10 to 14, wherein the total amount of rhodium is added in the first process step.

## Revendications

1. Procédé d'hydroformylation de 1-oléfines par réaction de celles-ci sur un catalyseur complexe contenant du rhodium comprenant un mélange de ligands complexes organiques contenant du phosphore, avec addition d'hydrogène et de monoxyde de carbone, **caractérisé en ce que** la réaction est effectuée dans une plage de pression supérieure ou égale à 0,5 MPa et inférieure ou égale à 5 MPa et dans un solvant choisi dans le groupe des solvants ayant un point d'ébullition supérieur ou égal à 180 °C et inférieur ou égal à 250 °C, avec une concentration en rhodium supérieure ou égale à 50 ppm et inférieure ou égale à 250 ppm en complexes catalytiques avec au moins deux ligands complexes différents choisis dans le groupe constitué par les arylphosphines et les tri-cycloalkylphosphines, la proportion de cycloalkylphosphines dans la quantité totale de ligands organophosphorés est supérieure ou égale à 1 % en moles et inférieure ou égale à 67 % en moles, et le rapport molaire ligands organophosphorés/rhodium, exprimé en quantité molaire de ligands organophosphorés divisée par la quantité molaire de rhodium, est inférieur ou égal à 85.

2. Procédé selon la revendication 1, dans lequel l'hydroformylation est réalisée dans une plage de température supérieure ou égale à 80°C et inférieure ou égale à 140°C.

3. Procédé selon l'une des revendications précédentes, dans lequel le rapport molaire des ligands arylphosphine aux ligands cycloalkylphosphine, exprimé comme quantité molaire de ligands arylphosphine divisée par la quantité molaire de ligands cycloalkylphosphine, est supérieur ou égal à 0,5 et inférieur ou égal à 75.

4. Procédé selon l'une des revendications précédentes, dans lequel le rapport molaire des ligands arylphosphine au rhodium est supérieur ou égal à 5 et inférieur ou égal à 75.

5. Procédé selon l'une des revendications précédentes, dans lequel le rapport molaire des ligands cycloalkylphosphine au rhodium est supérieur ou égal à 1 et inférieur ou égal à 10.

6. Procédé selon l'une des revendications précédentes, dans lequel l'arylphosphine est la triphénylphosphine.

7. Procédé selon l'une des revendications précédentes, dans lequel la cycloalkylphosphine est la tricyclohexylphosphine.

8. Procédé selon l'une des revendications précédentes, dans lequel la 1-oléfine est choisie dans le groupe constitué d'oléfines C3-C8 ou de mélanges de celles-ci.

9. Procédé selon l'une des revendications précédentes, dans lequel le rapport molaire de gaz de synthèse à 1-oléfine, exprimé comme (quantité molaire de H2 + quantité molaire de CO) divisée par la quantité molaire de 1-oléfine, est supérieur ou égal à 1:1 et inférieur ou égal à 5:1.

10. Utilisation du procédé selon l'une des revendications précédentes pour l'hydroformylation de 1-oléfines sur un catalyseur complexe, **caractérisé en ce que** l'hydroformylation est réalisée en deux étapes, la réaction est effectuée, au cours d'une première étape du procédé, en présence d'un catalyseur complexe contenant du rhodium, comprenant des ligands arylphosphine et sans ligands cycloalkylphosphine, dans un solvant choisi dans le groupe constitué par les alcools, les des acétals ou des alcanes ayant une longueur de chaîne supérieure ou égale à C10 ou leur mélange, et dans lequel, au cours d'une deuxième étape du procédé, un autre solvant ayant un point d'ébullition supérieur ou égal à 180°C et inférieur ou égal à 250°C et, en outre, des ligands tricycloalkylphosphine sont ajoutés au mélange réactionnel de la première étape du procédé.

11. Utilisation selon la revendication 10, dans laquelle le solvant de la deuxième étape de procédé est choisi dans le groupe constitué d'alcools ayant une longueur de chaîne supérieure ou égale à C8, d'acétals ayant une longueur de chaîne supérieure ou égale à C13, d'alcanes ayant une longueur de chaîne supérieure ou égale à C10, ou de mélanges d'au moins deux composants de ce groupe.

12. Utilisation selon l'une des revendications 10 ou 11, dans laquelle le rapport pondéral du solvant ajouté dans la deuxième étape de procédé à la quantité de solvant de la première étape de procédé est supérieur ou égal à 4 et inférieur ou égal à 20.

13. Utilisation selon l'une des revendications 10 à 12, dans laquelle la concentration des ligands arylphosphine dans la première étape de procédé, rapportée au poids total de la solution de procédé, est supérieure ou égale à 10 % en poids et inférieure ou égale à 30 % en poids.

14. Utilisation selon l'une des revendications 10 à 13, dans laquelle la concentration de la cycloalkylphosphine ajoutée dans la deuxième étape de procédé, rapportée au poids total de la solution de procédé, est supérieure ou égale à 0,01 % en poids et inférieure ou égale à 1 % en poids.

15. Utilisation selon l'une des revendications 10 à 14, dans laquelle la quantité totale de rhodium est ajoutée dans la première étape de procédé.
